# EUROPÄISCHE PATENTSCHRIFT

(11) **EP 2 338 561 B1**
(45) Veröffentlichungstag und Bekanntmachung des Hinweises auf die Patenterteilung: **25.03.2020**
(21) Anmeldenummer: 10192883.6
(22) Anmeldetag: 29.11.2010
(51) Int. Cl.: A61N 1/37

(54) **Implantierbares medizinisches Gerät mit verlängertem Noise-Mode**
Implantable medical device with an extended noise mode
Dispositif médical implantable avec un mode bruit prolongé

(30) Priorität: 22.12.2009 US 288862 P
(43) Veröffentlichungstag der Anmeldung: 29.06.2011
(73) Patentinhaber: BIOTRONIK SE & Co. KG, 12359 Berlin (DE)
(72) Erfinder: Dörr, Thomas, 12437 Berlin (DE); Weiss, Ingo, 12435 Berlin (DE)
(74) Vertreter: Keck, Hans-Georg

(56) Entgegenhaltungen:
- WO-A1-96/41203
- US-A1- 2006 167 496
- US-A1- 2006 293 591
- US-A1- 2007 203 523
- US-B1- 7 561 915

## Beschreibung

Die Erfindung bezieht sich auf eine Vorrichtung und ein Verfahren zum Umgang mit elektromagnetischen Feldern, speziell solche Felder, wie sie bei Kauteranwendungen auftreten.

Obwohl Kauteranwendungen einen immer höheren Stellenwert in der Medizin, besonders bei der chirurgischen Intervention und im Zusammenhang mit Gefäßverletzungen, erhalten, ist ein Teil der Patienten für Kauteranwendungen kontraindiziert. Eine solche Kontraindikation kann durch ein mindestens teilweise implantiertes medizinisches Gerät (im Folgenden auch Implantat oder IMD) gegeben sein, insbesondere bei sogenannten ICD- (Implanted Cardiverter Defribrilator) Patienten.

Bei Kauteranwendungen im Stand der Technik handelt es sich zumeist um elektrisches Kautern, das heißt, dass Kautern mit Hilfe eines durch Stromfluss erhitzten Objektes, zum Beispiel eines dünnen Drahtes, durchgeführt wird. Das Kautern wird auch als Diathermie bezeichnet. Man unterscheidet neben der elektrischen hochfrequenten Diathermie auch noch die Ultraschalldiathermie und die Mikrowellendiathermie. Als im Allgemeinen unkritisch für IMD-Träger gilt die Ultraschalldiathermie, während die Hochfrequenzdiathermie und die Mikrowellendiathermie mit einem IMD interferieren können.

Um Kauteranwendungen, speziell mittels der Hochfrequenzdiathermie und der Mikrowellendiathermie dennoch zu ermöglichen, sind vor allem asynchrone Betriebsarten für Schrittmacher und ICD Patienten bekannt. Diese haben den Nachteil, dass von der asynchronen Stimulation ein erhebliches proarrhythmisches Risiko ausgeht. Insbesondere bei ICD-Patienten ist eine unnötige asynchrone Stimulation kontraindiziert.

Derzeit bekannte Störerkennungen aller etablierten Schrittmacher (IPG)- und ICD-Systeme sind hierfür nicht anwendbar, da diese sofort nach Ende des Anliegens der Störung den asynchronen Störmode verlassen. Damit versagt diese bei gepulsten Störungen, wie sie bei unter anderem Kauteranwendungen auftreten.

US 20060293591 A1 beschreibt ein implantierbares medizinisches Gerät mit einer Telemetrie-Antenne und einem Lead mit einem länglichen Körper umfassend einen Leiter, der sich von einem proximalen Anschluss zu einer distalen Elektrode erstreckt.

US 7,561,915 B1 beschreibt ein Telemetriegerät welches in Kommunikation mit einem MRT System steht, wobei das Telemetriegerät dazu ausgelegt ist, mit einem implantierbaren medizinschen Gerät zu kommunizieren.

Aufgabe der Erfindung ist es, eine Vorrichtung und eine Methode für medizinische Geräte und implantierbare medizinische Geräte bereitzustellen, die die Nachteile des Stands der Technik beheben und einen sicheren Betrieb in Gegenwart von elektromagnetischen Störfeldern, speziell ausgehend von Kauteranwendungen, erlauben.

Die Aufgabe wird durch ein zumindest teilweise implantierbares medizinisches Gerät (IMD) mit den Merkmalen des Anspruchs 1 und dem Verfahren nach Anspruch 11 gelöst.

Dabei beinhaltete das zumindest teilweise implantierbare medizinische Gerät (IMD) mindestens: eine Einheit zur Detektion von elektromagnetischen Störfeldern, wobei die Einheit über mindestens einen Sensor und/oder Indikator für elektromagnetische Störfelder verfügt und/oder ausgelegt ist, nicht physiologische Signale zu detektieren und zu erkennen, mindestens eine Steuereinheit, die mit der Einheit zur Detektion von elektromagnetischen Störfeldern verbindbar ist, mindestens beinhaltend einen Zeitmesser und eine Detektionseinheit für elektrische Messgrößen und/oder mindestens beinhaltend eine Stimulationseinheit, mindestens eine Elektrodenleitung, die mit der Steuereinheit und/oder mit der Einheit zur Detektion von elektromagnetischen Störfeldern verbindbar ist und die am anderen Ende eine Elektrode aufweist, die im zumindest teilweise implantierten Zustand im Kontakt mit Körpergewebe bringbar ist und die sich entweder im Körperinneren erstreckt oder auf der Oberfläche eines Implantates befinden kann, wobei die Einheit zur Detektion von elektromagnetischen Störfeldern bei der Erkennung von nicht physiologischen Signalen und/oder elektromagnetischen Störfeldern für eine erste vorgebbare Zeit die detektierten Signale als nicht physiologische Signale bewertet und/oder die Stimulationseinheit für eine zweite vorgegebene Zeit in einem asynchronen Betriebszustand versetzt wird, in dem der Träger des IMD asynchron stimuliert wird.

Bevorzugt ist, dass das IMD ein implantierbarer oder externer Stimulator ist.
Dabei ist unter einem externen Stimulator zum Beispiel, aber nicht beschränkt auf, ein externer Herzstimulator, ein externer Defibrillator und/oder Neurostimulator zu verstehen, wobei bei externen Stimulatoren Teile implantiert oder temporär implantiert sein können.

Ebenfalls bevorzugt ist, dass das IMD ein Implantat zur Überwachung von Körperfunktionen ist, wie, aber nicht beschränkt auf, einen implantierbaren Herzmonitor. Ein solches Implantat kann über sich in das Körperinnere erstreckende Elektrodenleitungen mit mindestens einer Elektrode und/oder über mindestens eine Elektrode auf der Implantatsoberfläche zur Detektion von physiologischen Signalen verfügen.

Auch wird bevorzugt, dass der asynchrone Betriebszustand immer dann für die zweite vorgegebene Zeit aktiviert wird, wenn mindestens ein Stimulationsimpuls abgegeben wurde bei gleichzeitiger Erkennung von nicht physiologischen Signalen und/oder bei Erkennung von elektromagnetischen Störfeldern.

Des Weiteren wird bevorzugt, dass der asynchrone Betriebszustand immer dann für die zweite vorgegebene Zeit aktiviert wird, wenn eine vorbestimmbare Mindestanzahl von nicht physiologischen Signalen in einem vorbestimmbaren Zeitfenster detektiert wird.

Bevorzugt ist auch, dass der asynchrone Betriebszustand ein A00, V00 oder D00 Betriebszustand ist.

Ebenfalls bevorzugt wird, dass der asynchrone Betriebszustand eine biventrikuläre Stimulation einschließt.

Auch wird bevorzugt, dass der asynchrone Betriebszustand mit einer Änderung der Stimulationsfrequenz verbunden ist, wobei sich die asynchrone Stimulationsfrequenz aus der Herzfrequenz vor einer detektierten elektromagnetischen Störung und/oder der Detektion von nicht physiologischen Signalen berechnet.

Eine mögliche Berechnungsform der asynchronen Stimulationsfrequenz aus der Herzfrequenz vor einer detektierten elektromagnetischen Störung und/oder der Detektion von nicht physiologischen Signalen besteht in dem Hinzuaddieren von einer vorherbestimmbaren Anzahl von Herzschlägen pro Zeiteinheit, wie aber nicht beschränkt auf Herzfrequenz + 10 bpm.

Bevorzugt ist auch, dass detektierte Signale als nicht physiologische Signale bewertet werden und/oder der asynchrone Betriebszustand nur für eine vorgebbare erste Zeit durch die Erkennung von nicht physiologischen Signalen und/oder elektromagnetischen Störfeldern aktivierbar ist.

Besonders bevorzugt wird, dass die vorgebbare erste Zeit im Bereich von Stunden und/oder Tagen liegt.

Ebenfalls bevorzug wird, dass die Einheit zur Detektion von elektromagnetischen Störfeldern mindestens einen der folgenden Sensoren oder Indikatoren umfasst: GMR-Sensor, MagFET-Sensor, Hallsensor, elektrooptische Wandler als Indikator, die Überwachung von Batteriespannungen während Kondensatorladeprozessen als Indikator, die Detektion von RF-Feldern als Indikator, die Detektion von magnetischen Grandientenfeldern als Indikator, und die Detektion von durch elektromagnetische Felder induzierten Strömen als Indikator.

Gelöst wird die Aufgabe auch durch ein Verfahren zum Umgang mit von einem IMD detektierbaren elektromagnetischen nach Anspruch 11. Das Verfahren lässt sich beispielsweise mit jedem der obigen IMDs ausführen.

Einige Aspekte der Erfindung werden in den Figuren 1 bis 4 dargestellt.
- Fig. 1: Schematische Darstellung der Ausgangssituation bei gepulsten Störungen
- Fig. 2: Blockschaltbild einer erfindungsgemäßen Herzstimulators.
- Fig. 3: Schematische Darstellung der Steuerung des in Figur 2 dargestellten Modeumschalters.
- Fig. 4: Beispielhafte Situation mit erfindungsgemäßer Lösung.

In einem Beispiel wird das erfindungsgemäße System in einem Schrittmacher implementiert. Schrittmacherabhängige Patienten werden im Falle einer gepulsten Störung möglicherweise dauerhaft nicht stimuliert, da die herkömmliche Noiseerkennung bei ICD/IPG den Störmode sofort verlässt, wenn die Störung beendet wird. Um die Stimulation während einer Kauteranwendung jedoch zu gewährleisten, wird hier ein erweiterter Störmode implementiert, der einen zusätzlichen Timer umfasst, um so die Zeit einer asynchronen Stimulation nach einer einmaligen Störerkennung zu verlängern. Diese Zeit wird dabei an die typische Dauer der Prozedur per vorheriger Programmierung angepasst. Dieser Störmode kann zeitlich limitiert werden, wie, aber nicht beschränkt, auf Stunden, Tage oder Wochen.

In einem anderen Beispiel wird der erweiterte Störmode in einem Herzmonitor implementiert, der während einer gepulsten Störung möglicherweise dauerhaft keine Herzsignale aufzeichnet und/oder die identifizierten Störungen markiert, um eine Fehlinterpretation der aufgezeichneten Daten zu verhindern. Der Störmode wird erst dann wieder verlassen, wenn nach einer voreinstellbaren Zeit keine weiteren Störungen detektiert wurden.

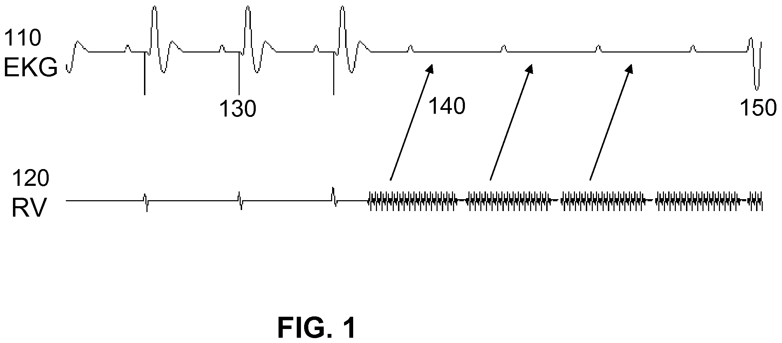

Figur 1 stellt eine mit dem Stand der Technik typische Situation dar. Hier wird die Stimulation eines Schrittmachers durch die gepulsten Störfelder des MRT fälschlicherweise inhibiert und damit eine Unterversorgung des Patienten herbeigeführt (100).

Dargestellt ist hier das Oberflächen-EKG (110) und das (optisch übertragene) intrakardiale rechtsventrikuläre Elektrogramm (120) eines Zweikammerschrittmachers bei einem Patienten mit totalem AV-Block.
Vor dem Einsetzen der MRT-Störung wird der Patient vorhofsynchron stimuliert (130). Diese Stimulationsimpulse werden jedoch durch die gepulste Störung (140) regelmäßig inhibiert. Diese wiederholte Inhibierung tritt auf, da die Störung immer wieder unterbrochen wird und der Schrittmacher daher nicht in einen Störmode schalten kann.
In diesem Beispiel hat der Patient noch einen sehr langsamen ventrikulären Ersatzrhythmus (150), so dass eine minimale Pumpfunktion des Ventrikels gegeben war. Ohne diesen Ersatzrhythmus wäre hier ein Herzstillstand eingetreten. Neben dieser Möglichkeit kann der Stimulator aber auch im Vorfeld von zum Beispiel einer Kauteranwendung in einen asynchronen Stimulationsmodus versetzt werden, was aber ein erhöhtes Risiko von Tachykardien birgt.

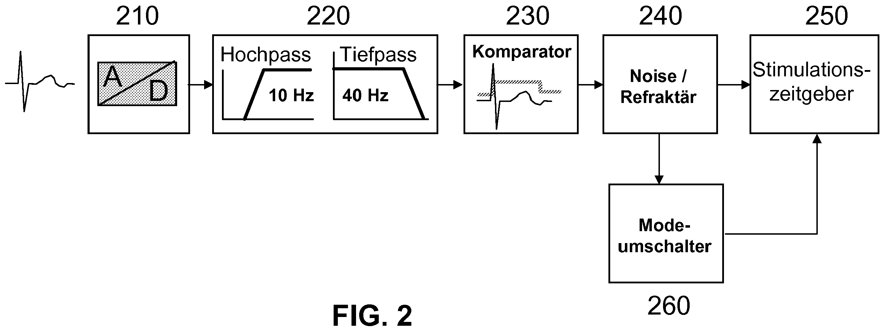

Die Figur 2 zeigt das Blockschaltbild eines erfindungsgemäß erweiterten Herzstimulators. Die IEGM-Signale werden zunächst verstärkt und digitalisiert (210) und anschließend in einer digitalen Filtereinheit (220) für die nachfolgende Signalverarbeitung konditioniert. In einer adaptiven Komparatorstufe (230) werden die Senseereignisse generiert und anschließend den Noise- und Refraktärgliedern (240) zugeführt. Die so bewerteten Signale werden dem Stimulationszeitgeber (250) zugeführt und mit dieser Information die Schrittmacherstimulation gesteuert.
Erfindungsgemäß umfasst das Blockschaltbild einen zusätzlichen Modeumschalter (260), der über eine Verbindung von der Noise-Bewertung (240) gesteuert wird und immer dann, wenn Noise-Ereignisse aufgetreten sind, den Stimulationszeitgeber (250) für eine programmierte Dauer in einen asynchronen Stimulationsmode (V00, D00) umschaltet.

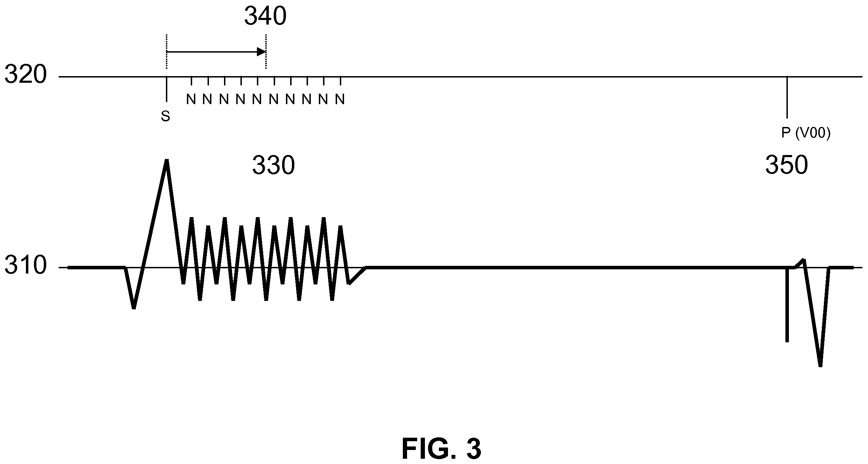

Die Figur 3 zeigt ein beispielhaftes Timing-Diagramm, das heißt die Figur 3 zeigt die Steuerung des in Figur 2 gezeigten Modeumschalters. Dargestellt sind der intrakardiale IEGM-Kanal (310) und der Markerkanal (320). In diesem Beispiel setzt eine Störung (330) nach einer wahrgenommenen R-Welle (S) ein. Im Markerkanal ist die Störung (330) anhand der Noise-Marker (N) erkennbar. Überschreitet die Dauer eines ununterbrochenen Noise-Zustandes eine einstellbare Zeit (340), dann signalisiert der Modeumschalter (260) dem Stimulationszeitgeber (250) eine Umschaltung in eine asynchrone Stimulation (350). Durch die Einführung der geforderten Noise-Dauer (340) kann zwischen Mehrfachtriggerung einer R-Welle (wird auch kurzzeitig als Noise bewertet) und einer relevanten gepulsten Störung unterschieden, die eine Modeumschaltung erforderlich macht.

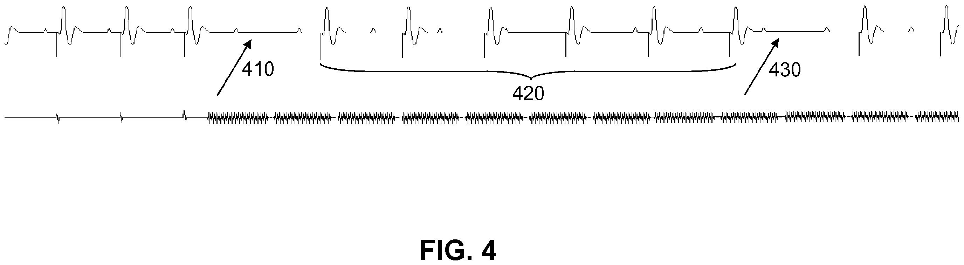

Die Figur 4 zeigt das in Figur 1 dargestellte Beispiel, nun jedoch mit der erfindungsgemäßen Störumschaltung. Auch hier wird die ventrikuläre Stimulation zunächst durch die einsetzende MRT-Störung inhibiert (410). Allerdings schaltet nun das Implantat automatisch für sechs Zyklen in einen programmierten Störmode, hier eine V00-Stimulation (420). Danach erfolgt automatisch die Umschaltung zurück in die ursprüngliche DDD-Betriebsart. Da die Störung noch vorhanden ist, wird eine weitere Stimulation inhibiert (430) und darauf hin wieder für sechs Zyklen in den V00-Mode geschaltet.
So ist es möglich, eine zuverlässige Back-up-Stimulation bei gepulsten Störungen zu realisieren, die den Patienten nicht für einen langen Zeitraum einer asynchronen Stimulation aussetzt.
Die Anzahl der Zyklen und/oder die Dauer der asynchronen Stimulation kann entsprechend variiert werden.

Erfindungsgemäße Implantate und Verfahren sind prinzipiell unabhängig von den gewählten Detektions- und Erkennungsmethoden für die gepulsten Störquellen. Allerdings werden für Implantate einerseits möglichst genaue Detektionsmethoden und andererseits ein möglichst geringer Energiebedarf bevorzugt. Damit ergeben sich als vorteilhafte Detektoren und/oder Erkennungsmöglichkeiten der Einsatz von GMR-Sensoren, MagFET-Sensoren, Hallsensoren, elektrooptische Wandlern, die Überwachung von Batteriespannungen während Kondensatorladeprozessen, die Detektion von RF-Feldern, die Detektion von magnetischen Grandientenfeldern, und die Detektion von durch elektromagnetische Felder induzierten Strömen. Bevorzugt wird die Technologie in Zusammenhang mit anderen Technologien, wie, aber nicht beschränkt auf, MRT-Erkennung (MRT = Magnetresonanztomographie) und MRT-sicherer Betrieb, implementiert um Synergieeffekte nutzen zu können. So können die Methoden und/oder Detektionsvorrichtungen für verschiedene Anwendungen genutzt werden.

Es wird somit ermöglicht, eine zuverlässige Stimulation auch bei gepulsten elektromagnetischen Störungen sicherzustellen, so dass Kauter-Anwendungen für Schrittmacher und ICD-Systeme eingesetzt werden können, ohne dass zuvor eine asynchrone Stimulationsbetriebsart programmiert werden muss. Auch sind andere Anwendungen mit gepulsten elektromagnetischen Störungen möglich, wie sie zum Beispiel bei dem Einsatz von elektromagnetischen Radio frequenzfeldern und/oder Gradientenfeldern auftreten.

## Patentansprüche

1. Zumindest teilweise implantierbares medizinisches Gerät (IMD), mit
- einer Einheit zur Detektion von elektromagnetischen Störfeldern, wobei die Einheit über mindestens einen Sensor und/oder Indikator für elektromagnetische Störfelder verfügt und/oder ausgelegt ist, nicht physiologische Signale zu detektieren und zu erkennen,
- mindestens einer Steuereinheit, die mit der Einheit zur Detektion von elektromagnetischen Störfeldern verbindbar ist, mindestens beinhaltend einen Zeitmesser und eine Detektionseinheit für elektrische Messgrößen und/oder mindestens beinhaltend eine Stimulationseinheit,
- mindestens einer Elektrodenleitung, die mit der Steuereinheit und/oder mit der Einheit zur Detektion von elektromagnetischen Störfeldern verbindbar ist und die am anderen Ende eine Elektrode aufweist, die im zumindest teilweise implantierten Zustand im Kontakt mit Körpergewebe bringbar ist und die sich entweder im Körperinneren erstreckt oder auf der Oberfläche eines Implantates befinden kann,
die Einheit zur Detektion von elektromagnetischen Störfeldern bei der Erkennung von nicht physiologischen Signalen und/oder elektromagnetischen Störfeldern für eine erste vorgebbare Zeit die detektierten Signale als nicht physiologische Signale bewertet und die Stimulationseinheit für eine zweite vorgegebene Zeit in einem asynchronen Betriebszustand versetzt wird, in dem der Träger des IMD asynchron stimuliert wird,
**dadurch gekennzeichnet, dass** der asynchrone Betriebszustand mit einer Änderung der Stimulationsamplitude und/oder der Stimulationspulsbreite verbunden ist.

2. IMD nach Anspruch 1, **dadurch gekennzeichnet, dass** das IMD ein implantierbarer oder externer Stimulator ist.

3. IMD nach Anspruch 1 oder 2, **dadurch gekennzeichnet, dass** der asynchrone Betriebszustand immer dann für die zweite vorgegebene Zeit aktiviert wird, wenn mindestens ein Stimulationsimpuls abgegeben wurde bei gleichzeitiger Erkennung von nicht physiologischen Signalen und/oder bei Erkennung von elektromagnetischen Störfeldern.

4. IMD nach einem der vorangegangenen Ansprüche, **dadurch gekennzeichnet, dass** der asynchrone Betriebszustand immer dann für die zweite vorgegebene Zeit aktiviert wird, wenn eine vorbestimmbare Mindestanzahl von nicht physiologischen Signalen in einem vorbestimmbaren Zeitfenster detektiert wird.

5. IMD nach einem der vorangegangenen Ansprüche, **dadurch gekennzeichnet, dass** der asynchrone Betriebszustand ein A00, V00 oder D00 Betriebszustand ist.

6. IMD nach einem der vorangegangenen Ansprüche, **dadurch gekennzeichnet, dass** der asynchrone Betriebszustand eine biventrikuläre Stimulation einschließt.

7. IMD nach einem der vorangegangenen Ansprüche, **dadurch gekennzeichnet, dass** der asynchrone Betriebszustand mit einer Änderung der Stimulationsfrequenz verbunden ist, wobei sich die asynchrone Stimulationsfrequenz aus der Herzfrequenz vor einer detektierten elektromagnetischen Störung und/oder der Detektion von nicht physiologischen Signalen berechnet.

8. IMD nach einem der vorangegangenen Ansprüche, **dadurch gekennzeichnet, dass** detektierte Signale als nicht physiologische Signale bewertet werden und/oder der asynchrone Betriebszustand nur für eine vorgebbare erste Zeit durch die Erkennung von nicht physiologische Signalen und/oder elektromagnetischen Störfeldern aktivierbar ist.

9. IMD nach Anspruch 8, **dadurch gekennzeichnet, dass** die vorgebbare erste Zeit im Bereich von Stunden und/oder Tagen liegt.

10. IMD nach einem der vorangegangenen Ansprüche, **dadurch gekennzeichnet, dass** die Einheit zur Detektion von elektromagnetischen Störfeldern mindestens einen der folgenden Sensoren oder Indikatoren umfasst:
- GMR-Sensor,
- MagFET-Sensor,
- Hallsensor,
- elektrooptische Wandler als Indikator,
- die Überwachung von Batteriespannungen während Kondensatorladeprozessen als Indikator,
- die Detektion von RF-Feldern als Indikator,
- die Detektion von magnetischen Grandientenfeldern als Indikator, und
- die Detektion von durch elektromagnetische Felder induzierten Strömen als Indikator.

## Claims

1. An at least partially implantable medical device (IMD) comprising
- a unit for detecting electromagnetic interference fields, wherein the unit has at least one sensor and/or indicator for electromagnetic interference fields and/or is designed to detect and identify non-physiological signals,
- at least one control unit, which is connectable to the unit for detecting electromagnetic interference fields, at least comprising a timer and a detection unit for electrical measured variables and/or at least comprising a stimulation unit,
- at least one electrode line which is connectable to the control unit and/or to the unit for detecting electromagnetic interference fields, and which at the other end has an electrode which in the at least partially implanted state may be brought into contact with bodily tissue, and which either extends inside a body or may be situated on the surface of an implant,
the unit for detecting electromagnetic interference fields, when identifying non-physiological signals and/or electromagnetic interference fields, evaluates the detected signals as non-physiological signals for a first specifiable time, and for a second specified time the stimulation unit is placed in an asynchronous operating state in which the wearer of the IMD is asynchronously stimulated,
**characterised in that** the asynchronous operating state is associated with a change in the stimulation amplitude and/or the stimulation pulse width.

2. The IMD according to claim 1, **characterised in that** the IMD is an implantable or external stimulator.

3. The IMD according to claim 1 or 2, **characterised in that** the asynchronous operating state is activated for the second specified time whenever at least one stimulation pulse has been emitted, with simultaneous identification of non-physiological signals and/or with identification of electromagnetic interference fields.

4. The IMD according to any one of the preceding claims, **characterised in that** the asynchronous operating state is activated for the second specified time whenever a predeterminable minimum number of non-physiological signals is detected in a predeterminable time window.

5. The IMD according to any one of the preceding claims, **characterised in that** the asynchronous operating state is an A00, V00, or D00 operating state.

6. The IMD according to any one of the preceding claims, **characterised in that** the asynchronous operating state includes a biventricular stimulation.

7. The IMD according to any one of the preceding claims, **characterised in that** the asynchronous operating state is associated with a change in the stimulation frequency, wherein the asynchronous stimulation frequency is calculated from a heart rate before a detected electromagnetic interference and/or detection of non-physiological signals.

8. The IMD according to any one of the preceding claims, **characterised in that** detected signals are evaluated as non-physiological signals, and/or the asynchronous operating state is activatable only for a specifiable first time by the identification of non-physiological signals and/or electromagnetic interference fields.

9. The IMD according to claim 8, **characterised in that** the specifiable first time lies in the range of hours and/or days.

10. The IMD according to any one of the preceding claims, **characterised in that** the unit for detecting electromagnetic interference fields comprises at least one of the following sensors or indicators:
- GMR sensor,
- MagFET sensor,
- Hall sensor,
- electro-optical converter ,as indicator,
- the monitoring of battery voltages during capacitor charging processes, as indicator,
- the detection of RF fields, as indicator,
- the detection of magnetic gradient fields, as indicator, and
- the detection of currents induced by electromagnetic fields, as indicator.

## Revendications

1. Appareil médical implantable (AMI) au moins partiellement, doté
- d'une unité de détection de champs d'interférences électromagnétiques, l'unité disposant d'au moins un capteur et/ou d'un indicateur de champs d'interférences électromagnétiques, et/ou étant conçu pour détecter et reconnaître des signaux non physiologiques,
- d'au moins une unité de commande qui peut être reliée avec l'unité de détection de champs d'interférences électromagnétiques, contenant au moins un chronomètre et une unité de détection de grandeurs de mesure électriques, et/ou contenant au moins une unité de stimulation,
- d'au moins une ligne d'électrode qui peut être reliée avec l'unité de détection de champs d'interférences électromagnétiques et qui présente une électrode à l'autre extrémité, laquelle peut être mise en contact avec du tissu corporel dans l'état au moins partiellement implanté et qui s'étend soit à l'intérieur du corps, soit peut se trouver sur la surface d'un implant,
l'unité de détection de champs d'interférences électromagnétiques, lors de la découverte de signaux non physiologiques, et/ou de champs d'interférences électromagnétiques, pour une première durée pouvant être définie, peut évaluer les signaux détectés comme des signaux non physiologiques et l'unité de stimulation peut être placée dans un état de fonctionnement asynchrone dans lequel le porteur de
l'AMI est stimulé de manière asynchrone pour une seconde durée prédéfinie, **caractérisé en ce que** l'état de fonctionnement asynchrone est relié à une modification de l'amplitude de stimulation et/ou à la largeur de l'impulsion de stimulation.

2. AMI selon la revendication 1, **caractérisé en ce que** l'AMI est un stimulateur implantable ou externe.

3. AMI selon la revendication 1 ou la revendication 2, **caractérisé en ce que** l'état de fonctionnement asynchrone est toujours activé pour la seconde durée prédéfinie lorsqu'au moins une impulsion de stimulation a été délivrée pour une découverte simultanée de signaux non physiologiques et/ou lors d'une découverte de champs d'interférences électromagnétiques.

4. AMI selon l'une des revendications précédentes, **caractérisé en ce que** l'état de fonctionnement asynchrone est toujours activé pour la seconde durée prédéfinie lorsqu'un nombre minimal pouvant être prédéfini de signaux non physiologiques est détecté dans un intervalle de temps pouvant être prédéfini.

5. AMI selon l'une des revendications précédentes, **caractérisé en ce que** l'état de fonctionnement asynchrone est un état de fonctionnement A00, V00 ou D00.

6. AMI selon l'une des revendications précédentes, **caractérisé en ce que** l'état de fonctionnement asynchrone englobe une stimulation biventriculaire.

7. AMI selon l'une des revendications précédentes, **caractérisé en ce que** l'état de fonctionnement asynchrone est relié à une modification de la fréquence de stimulation, la fréquence de stimulation asynchrone se calculant à partir de la fréquence cardiaque d'une interférence électromagnétique détectée et/ou de la détection de signaux non physiologiques.

8. AMI selon l'une des revendications précédentes, **caractérisé en ce que** des signaux détectés sont évalués comme des signaux non physiologiques et/ou l'état de fonctionnement asynchrone peut être activé uniquement pour une première durée pouvant être prédéfinie par la découverte de signaux non physiologiques et/ou de champs d'interférences électromagnétiques.

9. AMI selon la revendication 8, **caractérisé en ce que** la première durée pouvant être prédéfinie se situe dans une plage d'heures et/ou de jours.

10. AMI selon l'une des revendications précédentes, **caractérisé en ce que** l'unité de détection de champs d'interférences électromagnétiques comprend au moins un des détecteurs ou indicateurs suivants :
- un capteur de type magnétorésistance géante GMR,
- un capteur de type transistor MagFET,
- un capteur à effet Hall,
- un convertisseur électro-optique servant d'indicateur,
- la surveillance de tensions de batterie pendant des processus de charge d'un condensateur servant d'indicateur,
- la détection de champs de radiofréquences RF servant d'indicateur,
- la détection de champs de gradients magnétiques servant d'indicateur, et
- la détection de flux induits par des champs électromagnétiques servant d'indicateur.
